# EUROPEAN PATENT SPECIFICATION

(11) **EP 3 487 582 B1**
(45) Date of publication and mention of the grant of the patent: **25.11.2020**
(21) Application number: 17831651.9
(22) Date of filing: 18.07.2017
(51) Int. Cl.: A61N 5/06

(54) **OPTO-ACOUSTIC SELECTIVE MECHANICAL STIMULATION OF THE VESTIBULAR SYSTEM**
OPTOAKUSTISCHE SELEKTIVE MECHANISCHE STIMULATION DES VESTIBULÄREN SYSTEMS
STIMULATION MÉCANIQUE SÉLECTIVE OPTO-ACOUSTIQUE DU SYSTÈME VESTIBULAIRE

(30) Priority: 19.07.2016 US 201662363872 P
(43) Date of publication of application: 29.05.2019
(73) Proprietor: MED-EL Elektromedizinische Geräte GmbH, 6020 Innsbruck (AT)
(72) Inventor: SABA, Rami, 6020 Innsbruck (AT); SHAH, Darshan, 60528 Frankfurt am Main (DE); HÜBNER, Patrick, 6020 Innsbruck (AT); DEAS, Ross, 6020 Innsbruck (AT)
(74) Representative: Lucke, Andreas
(86) International application number: PCT/US2017/042487
(87) International publication number: WO 2018/017515

(56) References cited:
- WO-A1-2012/103271
- WO-A1-2017/142842
- US-A1- 2006 161 227
- US-A1- 2007 060 983
- US-A1- 2007 060 983
- US-A1- 2011 082 519
- US-A1- 2013 023 963
- US-A1- 2013 122 564
- US-A1- 2013 144 354
- US-B1- 6 379 325
- US-B1- 8 012 189
- US-B1- 8 012 189

## Description

### TECHNICAL FIELD

The present invention relates to implant systems and electrode array arrangements for treatment of partial vestibular disorders.

### BACKGROUND ART

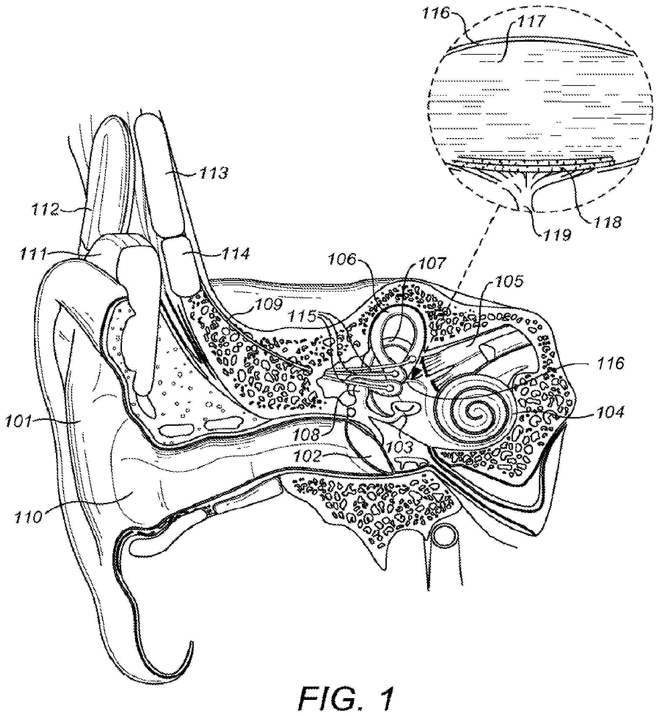

A normal ear directs sounds as shown in Figure 1 from the outer ear pinna **101** through the generally cylindrical ear canal **110** to vibrate the tympanic membrane **102** (eardrum). The tympanic membrane **102** moves the bones of the middle ear **103** (malleus, incus, and stapes) that vibrate the cochlea **104,** which in turn functions as a transducer to generate electric pulses to the brain that are interpreted as sounds.

The balance sensing functionality of the brain also is developed based on neural signals from the vestibular structures of the inner ear, one on each lateral side of the body. The balance sensing vestibular system involves the vestibular labyrinth, its three interconnected and mutually orthogonal semi-circular canals: the superior (anterior) canal **106,** posterior canal **107,** and horizontal (lateral) canal **108**-which sense rotational movement, as well as the macular organs **116** in the utricle and saccule, which sense linear movement. The canals **106, 107, 108** and the otolith organs **116** of the vestibular labyrinth contain hair cells **118** in a viscous endolymph **117** that sense head orientation and head movements, thereby activating vestibular nerve fibers **119** that send an electrical balance signal to the brain **105.**

When the head is stationary, the vestibular system generates neural activity at a certain rate that is transmitted by the vestibular nerve to the brain. When the head moves in a given direction, the vestibular system changes the neural activity rate on the affected nerve branch of the vestibular nerve which correlates with the head movement. Unfortunately some people suffer from damaged or impaired vestibular systems or from various diseases that affect intact vestibular systems such as Meniere's disease. Dysfunction of the vestibular system can cause problems such as unsteadiness, vertigo (feeling of rotation) and unsteady vision. To treat such problems, electrical stimulation of the vestibular system can help to restore the balancing function, and vestibular implants are currently under development to provide such an artificial balance signals.

Fig. 1 also shows some components of a vestibular implant system such as is described in U.S. Patent 8,751,012. An external movement signal (from one or more sensors not shown) is processed by an external processor **111** to produce a vestibular stimulation signal. An external transmitter coil **112** couples the stimulation signal through the skin to an implanted receiver coil **113.** Implanted vestibular stimulator **114** than delivers the stimulation signal through an electrode lead **109** to vestibular stimulator electrodes **115** that electrically stimulate target neural tissue such as the semicircular canals **106, 107, 108,** one or both otolith organs, and/or the vestibular nerve **105** or ganglion for vestibular sensation by the patient as a balance signal.

U.S. Patent 7,488,341 and U.S. Patent Publication 2007/0100263 describe invasive vestibular implant systems using an actuator based on piezoelectric material, an inflatable balloon, or a piston to mechanically stimulate the membranous labyrinth and thereby generate pressure waves in the endolymphatic space. Those references also disclose a non-invasive mechanical stimulation of the vestibular system where an actuator is placed on the endosteum layer adjacent to the perilymphatic space, and the endosteum is mechanically stimulated to create pressure waves within the vestibular lumen. While that is not invasive, the pressure exerted without rupturing the endosteum may not be enough to successfully stimulate the macular organs.

The mechanical actuator arrangements suggested in U.S. Patent 7,488,341 and U.S. Patent Publication 2007/0100263 all imply moving parts, which can be prone to damage and failure, and also can be damaging to the delicate structures of the inner ear. In addition, mechanical stimulation by a single actuator is limited to eliciting selective mechanical stimulation of the ampullar sensory structures as well as the possibility of a global response of the vestibular system. But this cannot, however, selectively stimulate the macular organs in the sense of inter-selective stimulation between the utricle and saccule, as well as intra-selectively within a given macular organ (e.g. saccule).

For total bilateral vestibular loss, U.S. Patent Publication 20150039057 describes using electrical stimulation by placing an array of electrodes within a macular organ and providing selective stimulation. But electrical current spread is generally non selective and will inherently and unintentionally stimulate non-target neuron populations in the macular organ.
WO 2012/103271 A1 discloses an implantable stimulation device for which includes an implantable stimulation source carrier for insertion into or adjacent to target tissue. The stimulation source carrier includes stimulation contacts for delivering neural stimulation signals to nearby target tissue. At least one of the stimulation contacts is an optical stimulation element having multiple individual optical stimulation sub-elements for delivering optical stimulation signals to the nearby target tissue with controlled shape and direction.
US 2007/0060983 A1 discloses an apparatus to stimulate the vestibular system of an individual. The apparatus comprises an optical stimulator configured to optically stimulate a nerve area affecting a person's balance, and a control module coupled to the optical stimulator, the control module being configured to control the optical stimulator.

### SUMMARY

Embodiments of the present disclosure are directed to an implantable vestibular prosthesis system and method. The invention is as defined in the appended claims. Embodiments disclosed herein which do not fall under the scope of the claims, in particular methods, are provided for illustrative purposes only. An implantable optical array of optical sources is configured for engagement with a disordered vestibular system to deliver optical stimulation signals to target stimulation locations within the bony or membranous labyrinth of the disordered vestibular system. An implantable stimulation processor is connected to the optical array and configured to produce sequences of optical stimulation signals with the optical sources so as to generate directional pressure waves within the endolymphatic fluid directed to the target stimulation locations for vestibular perception by residual vestibular functioning.

The optical array may specifically be configured for placement against an outer surface of vestibular bone to deliver the optical stimulation signals by optical transmission through the vestibular bone, or for placement within a perilymphatic space outside the membraneous labyrinth, or for placement within the endolymphatic space within the membraneous labyrinth without mixing perilymph and endolymph.

The one or more target stimulation locations may be within a semi-circular canal, the utricle, or the saccule, and may include target stereocilia. The optical array may be a linear array, a two dimensional array, or a circular array of optical sources, which may specifically be vertical-cavity surface-emitting lasers (VCSELs). The optical stimulation signals may include optical pulses.

### BRIEF DESCRIPTION OF THE DRAWINGS

Figure 1 shows anatomical detail of a human ear implanted with a vestibular implant system.
Figure 2 shows a side cross-sectional view of an opto-acoustic vestibular stimulation electrode according to an embodiment of the present invention.
Figure 3 shows principles of a circular array according to an embodiment of the present invention.
Figure 4 shows principles of a two dimensional array according to an embodiment of the present invention.
Figure 5 shows locations at the vestibular system for placement of the implantable optical array according to an embodiment of the invention.

### DETAILED DESCRIPTION

Embodiments of the present invention are directed to an atraumatic mechanical vestibular stimulator with no moving parts, which can be used non-invasively and can selectively stimulate the macular organs using the optoacoustic effect that arises from optical stimulation. Optical stimulation with multiple optical sources using short focused pulses at a specific rate, energy and peak power can heat the target tissue so that the expansion due to the heat (overcoming thermal-stress confinement) generates a pressure wave. Such a pressure wave, like with electrical current spread, is omnidirectional from the source and can be used to mechanically stimulate sensory epithelium in the vestibular system. Such systems can be beneficial for patients having partial vestibular disorders with some residual hair cells and stereocilia.

For effective mechanical stimulation of the endolymphatic fluids within the membranous labyrinth with existing approaches (see, e.g., U.S. Patent 7,488,341 and U.S. Patent Publication 2007/0100263) imply an opening into the vestibular interior in order to directly stimulate the membranous labyrinth. By contrast, optical sources can be placed outside the vestibular canal, and provided that the bone is thin enough (either natural or with careful surgical preparation to preserve the integrity of the canal), the optical stimulation signals can be delivered via optical transmission through the bone to locations within the bony or membranous labyrinth of a partially disordered vestibular system to generate a directional pressure wave within the endolymphatic fluid for vestibular perception via residual vestibular functioning. In case of thicker bone the stimulation location may be for example within the bony labyrinth. In this case the directional pressure wave directly generated by the optical stimulation signal is within the perilymphatic fluid. This pressure wave deflects the soft membrane tissue between bony and membranous labyrinth that in turn displaces endolymphatic fluid such that a directional pressure wave in the endolymphatic fluid is indirectly generated. This new non-invasive stimulation solution needs no moving parts, in contrast to existing non-invasive ideas where the actuator acts directly on the endosteum with the risk of rupturing the membrane and consequent loss in residual function.

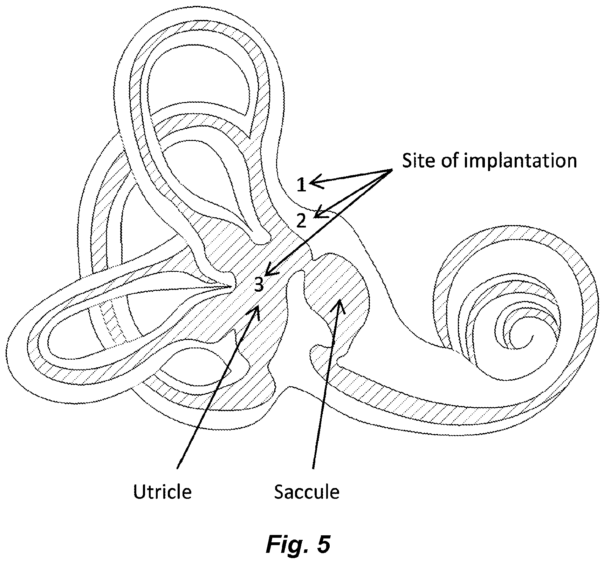

For example, an implantable vestibular prosthesis system and method can be implemented in an implantable vestibular stimulator **114** as shown in Figure 1 that provides by a stimulation processor control signals to the optical sources **115** on an implantable array that is configured for placement in a multitude of possible locations including: against or outside of an outer surface of vestibular bone outside of the fluidic spaces, for example where the bone is thin enough to allow light to shine through; against or within the inner surface of the vestibular bone within the perilymphatic space but not within the membranous labyrinth; within the membranous endolymphatic fluid spaces without causing damage resulting in the mixing of the perilymph and endolymph fluids, and the optical sources **115** are configured to deliver optical stimulation signals via optical transmission through the vestibular bone and/or vestibular fluids, i.e. endolymphatic or perilymphatic fluids, to stimulation locations in the membranous or bony labyrinth of a partially disordered vestibular system to generate a directional pressure wave within the endolymphatic fluid for vestibular perception via residual vestibular functioning, for example in the semi-circular canals **(106,107,108)** and/or otolith organs **116.** Some exemplary locations are shown in Figure 5 and numbered from 1 to 3. In one embodiment, an optical signal generation process (e.g., a software process running on a stimulation processor within the vestibular stimulator **114**) controls the optical sources **115** to produce the optical stimulation signals as sequences of optical pulses to generate directional pressure waves in the vestibular fluid that are directed to one or more target sensory epithelium locations within the disordered vestibular system for vestibular perception via residual vestibular functioning. In one embodiment the optical sources **115** may include converging lenses so as to focus the optical stimulation signal within the bony or membranous labyrinth, for example within the perilymphatic or endolymphatic fluid.

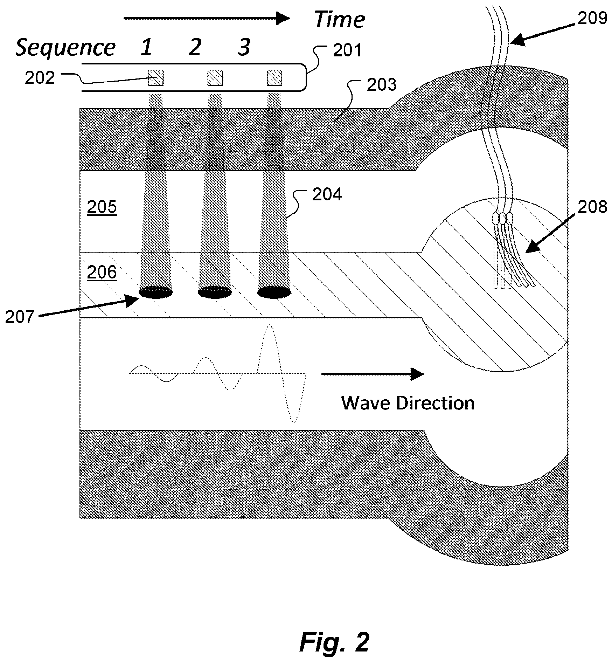

Stimulation with a single optical source would achieve the same omnidirectional result as with prior mechanical systems. However, if multiple optical sources **202** are placed in an optical array **201** as shown in Figure 2, then the optical stimulation signals **204** can be delivered through the vestibular bone **203** and the perilymph **205** to target locations **207** within the endolymph **206** and stimulated sequentially over time in a specified direction. Once the first optical source **202** is stimulated, a first omnidirectional pressure wave is generated at the target location **207** within the endolymph **206.** As the initial pressure wave passes the second target location **207** of the second optical source **202,** then the second optical source **202** is stimulated and a second pressure wave is generated that sums with the first pressure wave so that the summing of the resulting pressure waves in the direction of sequential stimulation encourages the endolymph **206** to move in that direction towards the target stereocilia **208** to cause them to deflect and generate neural signals to the vestibular nerve **209.** Focusing of the optical stimulation signals **204** at the target locations **207** can be enhanced by using focusing lenses, which would focus the energy at the target location, i.e. the optical stimulation signal heats up and expands the fluid only in the area of focus and allows for better control of pressure wave generation, allowing for a higher resolution of target locations. In addition, focusing the optical stimulation signals **204** at the target locations **207** may reduce the energy required for driving the optical source **202** to generate the same desired magnitude of the pressure wave as that without focusing. Although the target locations **207** are shown in the membranous labyrinth, it is understood and as described before, that they may be within the perilymph **205** in the bony labyrinth.

Such arrangements allow a more distant placement of the optical array **201** from the target stereocilia **208,** while providing direction-specific stimulation. This differs from other existing approaches that employ direct optical stimulation where attempting such directionality would pose greater risks of implantation trauma and would be prone to failure because of the many moving parts.

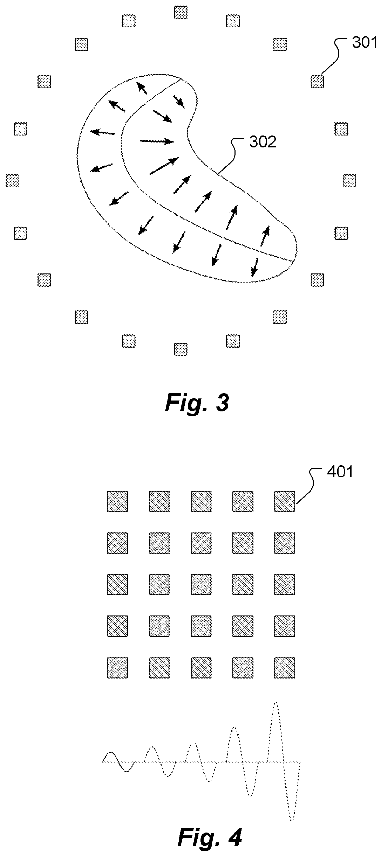

Selectivity is also an issue with regards to the utricle and saccule. In the case of electrical stimulation, given enough power, electrical current spread will stimulate any neuron in the vicinity regardless of the direction of current spread movement. Conversely, in the case of mechanical stimulation, it is the hair cells that respond to the stimulus, and hair cells and their stereocilia are typically organized and aligned in a particular way such that they are sensitive to movement in a particular plane and direction. For example, in the ampulla of a semi-circular canal, all the hair cells are aligned so that they are only sensitive to movement in the plane of that canal. In that case, one stimulation source would be enough (as with electrical stimulation), assuming that the stimulus signal does not extend to other ampulla. In the case of the utricle and saccule however, the hair cells and stereocilia are arranged in a multidirectional pattern such that their sensitivity to direction varies with position (as indicated by the arrows in Figure 3). In that case, placing a single stimulation source in an arbitrary position will generate a pressure wave that approaches the hair cells and stereocilia, but only those hair cells and stereocilia aligned in that direction will elicit a stimulus in the nerve. Due to the multidirectional alignment organization of the hair cells and stereocilia, this one single stimulation source lacks directionality. Instead, an array of multiple stimulation sources can be used in order to control which hair cells should respond (corresponding to the movement of the head). In that case, one or more sources would be stimulated simultaneously and/or sequentially in order to achieve the desired net direction and amplitude of movement of the vestibular fluid due to the pressure wave, and consequently deflecting the target stereocilia that are aligned in that specific direction.

Such a multi-source array could be placed either within the membranous labyrinth (invasive), in the perilymphatic space, or outside of the vestibule altogether (both non-invasive), provided that the bone in between the optical sources and the target locations is thin enough to allow enough light to pass through. As described before, the optical sources may include converging lenses so as to focus the optical stimulation signal within the bony or membranous labyrinth, for example within the perilymphatic or endolymphatic fluid.

Figure 3 shows an example of a linear array of multiple optical sources 301 arranged circularly around a macular organ **302.** In such an array, the pressure wave summation effects would result in multiple desired directions being simultaneously excited. By contrast, a two dimensional array of optical sources **401** as shown in Figure 4, would produce pressure wave summation effects resulting in directionality in a single direction as shown in Figure 2.

In specific embodiments, the optical sources may specifically be a vertical-cavity surface-emitting lasers (VCSELs), LEDs, or optical fibers. In the case of VCSELs, sequential stimulation avoids overuse of the VCSELs and so reduces the risk of overheating and failure. Sequential VCSEL stimulation also suggests reduced power requirements per VCSEL, which then further suggests that smaller size VCSELs could be used, with less heat produced, and so reduced cooling time needed.

It will be understood that for placement of the optical sources on the surface of vestibular bone, that bone needs to be thin enough to allow enough light to pass through. Embodiments will be useful only when hair cells and stereocilia retain at least some residual vestibular function, so only patients with partial vestibular loss may benefit. The optical sources may lose efficiency over time and their output levels may be monitored and the control signal adjusted accordingly.

In the foregoing, references to vestibular implant systems should be understood broadly to include all implantable arrangements that provide stimulation signals affecting the balance sensing system. Specifically such arrangements may or may not include motion sensors, whether internal or external. For example, a vestibular implant system without motion sensing signals may be useful for treatment related to Meniere's disease and may be thought of as a Meniere's implant. And vestibular implant arrangements may also be integrated together with other related implantable systems such as middle ear implants, cochlear implants, bone conduction implants, auditory brainstem implants, etc.

Although various exemplary embodiments of the invention have been disclosed, it should be apparent to those skilled in the art that various changes and modifications can be made which will achieve some of the advantages of the invention without departing from the true scope of the invention, which is defined by the appended claims.

## Claims

1. An implantable vestibular prosthesis system (114) for mechanically stimulating sensory epithelium in a vestibular system using an opto-acoustic effect arising from optical stimulation comprising:
an implantable optical array (201, 301, 401) including a plurality of optical sources (202) configured for engagement with a disordered vestibular system to deliver optical stimulation signals (204) to target optical stimulation locations (207) within the bony or membranous labyrinth of the disordered vestibular system; and
an implantable stimulation processor connected to the optical array (201) and configured for carrying out an optical signal generating process controlling the optical sources (202) to produce the optical stimulation signals (204) as sequences of optical pulses to produce the optical stimulation signals (204) with the optical sources so as to generate directional pressure waves within the endolymphatic fluid directed to target stimulation locations (208) for vestibular perception by residual vestibular functioning.

2. The system according to claim 1, wherein the optical array (201) is configured for placement against an outer surface of vestibular bone (203) to deliver the optical stimulation signals (204) by optical transmission through the vestibular bone (203).

3. The system according to claim 1, wherein the optical array (201) is configured for placement within a perilymphatic space outside the membraneous labyrinth.

4. The system according to claim 1, wherein the optical array (201) is configured for placement within the membraneous labyrinth without mixing perilymph and endolymph.

5. The system according to claim 1, wherein the one or more target stimulation locations (208) include stereocilia of the disordered vestibular system.

6. The system according to claim 1, wherein the one or more target stimulation locations are within a semi-circular canal of the disordered vestibular system.

7. The system according to claim 1, wherein the one or more target stimulation locations are within the utricle of the disordered vestibular system.

8. The system according to claim 1, wherein the one or more target stimulation locations are within the saccule of the disordered vestibular system.

9. The system according to claim 1, wherein the optical array (201) is a linear array of optical sources.

10. The system according to claim 1, wherein the optical array is a two dimensional array (401) of optical sources.

11. The system according to claim 1, wherein the optical array is a circular array (301) of optical sources.

## Patentansprüche

1. Implantierbares vestibuläres Prothesensystem (114) zur mechanischen Stimulierung des sensorischen Epithels in einem vestibulären System unter Verwendung eines optoakustischen Effekts, der sich aus der optischen Stimulation ergibt, umfassend:
ein implantierbares optisches Array (201, 301, 401) mit einer Vielzahl von optischen Quellen (202), die dazu konfiguriert sind, in ein gestörtes vestibuläres System einzugreifen, um optische Stimulationssignale (204) an Zielstellen (207) der optischen Stimulation innerhalb des knöchernen oder membranösen Labyrinths des gestörten vestibulären Systems zu liefern; und
einen implantierbaren Stimulationsprozessor, der mit dem optischen Array (201) verbunden und dazu konfiguriert ist, einen optischen Signalerzeugungsprozess auszuführen, der die optischen Quellen (202) steuert, um die optischen Stimulationssignale (204) als Sequenzen optischer Pulse zu erzeugen, um die optischen Stimulationssignale (204) mit den optischen Quellen zu erzeugen, so dass gerichtete Druckwellen innerhalb des endolymphatischen Fluids erzeugt werden, die auf Zielstimulationsorte (208) für die vestibuläre Wahrnehmung durch die vestibuläre Restfunktion gerichtet sind.

2. System nach Anspruch 1, wobei das optische Array (201) zur Platzierung gegen eine äußere Oberfläche des vestibulären Knochens (203) konfiguriert ist, um die optischen Stimulationssignale (204) durch optische Übertragung durch den vestibulären Knochen (203) hindurch zu liefern.

3. System nach Anspruch 1, wobei das optische Array (201) dazu konfiguriert ist, in einem perilymphatischen Raum außerhalb des Membranlabyrinths angeordnet zu werden.

4. System nach Anspruch 1, wobei das optische Array (201) dazu konfiguriert ist, innerhalb des Membranlabyrinths platziert zu werden, ohne Perilymphe und Endolymphe zu vermischen.

5. System nach Anspruch 1, wobei die eine oder die mehreren Zielstimulationsorte (208) Stereozilien des gestörten vestibulären Systems umfassen.

6. System nach Anspruch 1, wobei die eine oder die mehreren Zielstimulationsorte innerhalb eines halbkreisförmigen Kanals des gestörten vestibulären Systems liegen.

7. System nach Anspruch 1, wobei die eine oder die mehreren Zielstimulationsorte innerhalb des Utrikels des gestörten vestibulären Systems liegen.

8. System nach Anspruch 1, wobei die eine oder die mehreren Zielstimulationsorte innerhalb des Sakkulums des gestörten vestibulären Systems liegen.

9. System nach Anspruch 1, wobei das optische Array (201) ein lineares Array von optischen Quellen ist.

10. System nach Anspruch 1, wobei das optische Array ein zweidimensionales Array (401) von optischen Quellen ist.

11. System nach Anspruch 1, wobei das optische Array ein kreisförmiges Array (301) von optischen Quellen ist.

## Revendications

1. Système de prothèse vestibulaire implantable (114) destiné à la stimulation mécanique de l'épithélium sensoriel dans un système vestibulaire utilisant un effet opto-acoustique provenant d'une stimulation optique comprenant :
un réseau optique implantable (201, 301, 401) incluant une pluralité de sources optiques (202) conçues pour entrer en contact avec un système vestibulaire présentant un trouble pour délivrer des signaux de stimulation optique (204) à des emplacements de stimulation optique cibles (207) à l'intérieur du labyrinthe osseux ou membranaire du système vestibulaire présentant un trouble ; et
un processeur de stimulation implantable relié au réseau optique (201) et conçu pour réaliser un processus de génération de signaux optiques contrôlant les sources optiques (202) pour produire les signaux de stimulation optique (204) sous forme de séquences d'impulsions optiques pour produire les signaux de stimulation optique (204) avec les sources optiques de sorte à générer des ondes de pression directionnelles à l'intérieur du fluide endolymphatique dirigées vers des emplacements de stimulation cibles (208) en vue d'une perception vestibulaire par le fonctionnement vestibulaire résiduel.

2. Système selon la revendication 1, dans lequel le réseau optique (201) est conçu pour être placé contre une surface externe d'un os vestibulaire (203) pour délivrer les signaux de stimulation optique (204) par transmission optique à travers l'os vestibulaire (203).

3. Système selon la revendication 1, dans lequel le réseau optique (201) est conçu pour être placé à l'intérieur d'un espace périlymphatique à l'extérieur du labyrinthe membranaire.

4. Système selon la revendication 1, dans lequel le réseau optique (201) est conçu pour être placé à l'intérieur du labyrinthe membranaire sans mélange de la périlymphe et de l'endolymphe.

5. Système selon la revendication 1, dans lequel les un ou plusieurs emplacements de stimulation cibles (208) incluent un stéréocil du système vestibulaire présentant un trouble.

6. Système selon la revendication 1, dans lequel les un ou plusieurs emplacements de stimulation cibles sont à l'intérieur d'un canal semi-circulaire du système vestibulaire présentant un trouble.

7. Système selon la revendication 1, dans lequel les un ou plusieurs emplacements de stimulation cibles sont à l'intérieur de l'utricule du système vestibulaire présentant un trouble.

8. Système selon la revendication 1, dans lequel les un ou plusieurs emplacements de stimulation cibles sont à l'intérieur du saccule du système vestibulaire présentant un trouble.

9. Système selon la revendication 1, dans lequel le réseau optique (201) est un réseau linéaire de sources optiques.

10. Système selon la revendication 1, dans lequel le réseau optique est un réseau bidimensionnel (401) de sources optiques.

11. Système selon la revendication 1, dans lequel le réseau optique est un réseau circulaire (301) de sources optiques.
